# EUROPEAN PATENT APPLICATION

(11) **EP 4 850 692 A1**
(43) Date of publication of application: **09.12.2026**
(21) Application number: 24921104.6
(22) Date of filing: 06.02.2024
(51) Int. Cl.: A61K 45/00, A61P 13/12, C07C 321/28, C07D 233/72, C07D 249/12

(54) **USE OF PPAR?alpha/delta DUAL AGONIST IN TREATMENT OF POLYCYSTIC KIDNEY DISEASE**

(30) Priority: 30.01.2024 CN 202410127658
(71) Applicant: Harbin Medisan Pharmaceutical Co., LTD., Harbin, Heilongjiang 150025 (CN)
(72) Inventor: QIN, Zhen, Beijing Road, Limin Economic Development Zone, Hulan District Harbin, Heilongjiang 150025 (CN); GUO, Dong, Beijing Road, Limin Economic Development Zone, Hulan District Harbin, Heilongjiang 150025 (CN); LI, Quan, Beijing Road, Limin Economic Development Zone, Hulan District Harbin, Heilongjiang 150025 (CN); ZHAO, Wenchao, Beijing Road, Limin Economic Development Zone, Hulan District Harbin, Heilongjiang 150025 (CN); HE, Yichao, Beijing Road, Limin Economic Development Zone, Hulan District Harbin, Heilongjiang 150025 (CN); LI, Guihong, Beijing Road, Limin Economic Development Zone, Hulan District Harbin, Heilongjiang 150025 (CN); ZHANG, Daoxu, Beijing Road, Limin Economic Development Zone, Hulan District Harbin, Heilongjiang 150025 (CN); HU, Yuqing, Beijing Road, Limin Economic Development Zone, Hulan District Harbin, Heilongjiang 150025 (CN)
(74) Representative: Murgitroyd & Company
(86) International application number: PCT/CN2024/076206
(87) International publication number: WO 2025/161058

(57) **Abstract**

The use of a PPARα/δ dual agonist in the treatment of polycystic kidney disease. In an acute ADPKD mouse administration model, compared with a model group and a tolvaptan (TVP) control group, mice which received treatment using dual-receptor agonist compounds, such as DA002S, DA002S/V1 and GFT505, exhibited a reduction in kidney volume and a significantly decreased kidney weight index; moreover, after administration, the mice exhibited significantly reduced renal cysticity index (cyst area), suggesting that cyst development is inhibited. The *in-vivo* efficacy of DA002S and DA002S/V1 is superior to that of tolvaptan (TVP) at the same dose, thus indicating that the PPARα/δ dual agonist compound can inhibit the progression of polycystic kidney disease, and has good *in-vivo* efficacy for polycystic kidney disease.

## Description

### TECHNICAL FIELD

The present disclosure relates to the field of biomedicine, and in particular to use of a PPARα/δ dual agonist in the treatment of polycystic kidney disease.

### BACKGROUND

Peroxisome proliferator-activated receptors (PPARs), members of the type II hormone receptor superfamily, were first identified in 1990. In essence, they are a class of ligand-regulated gene transcription factors and are divided into three subtypes: α, δ (β), and γ (γ1, γ2). Upon binding to an agonist, PPARs form a heterodimer with retinoid X receptor (RXR), and bind to a peroxisome proliferator response element (PPRE), thereby initiating the transcription of downstream target genes.

Polycystic kidney disease, abbreviated to PKD, is also known as Potter (I) syndrome, Perlmann syndrome, congenital cystic kidney disease, cystic kidney, bilateral renal hypoplasia syndrome, benign multilocular cystic kidney nephroma, and polycystic disease. Polycystic kidney disease has two types: autosomal recessive (infantile) polycystic kidney disease (ARPKD) that occurs during infancy and is relatively rare clinically; and autosomal dominant (adult) polycystic kidney disease (ADPKD), also known as an adult type, which is commonly identified during adolescence or adulthood and may also occur at any age. The gene mutation loci related to PKD are shown below:

| S/N | OMIM database accession number | Gene name | Associated disease |
|---|---|---|---|
| I | 601313 | PKD1 | Adult ADPKD |
| II | 173910 | PKD2 | Adult ADPKD |
| III | 606702 | PKHD1 | Toddler-onset ARPKD |
| IV | 605284 | TSC1 | ADPKD |
| V | 191092 | TSC2 | ADPKD |
| VI | 605416 | DKK3 | ADPKD |

At present, neither treatment methods nor drugs for PKD can completely control disease progression, and the medical outcome thereof is kidney transplantation or death due to renal failure. Currently, the only drug approved by the European Union and the FDA for delaying progression of PKD is the vasopressin V2 receptor antagonist tolvaptan. The indication of drug is for treatment of clinically significant hypervolemic or euvolemic hyponatremia (concentration of serum sodium <125 mEq/L, or less marked but symptomatic and inadequately responsive to fluid restriction therapy), including patients with heart failure, cirrhosis, or syndrome of inappropriate antidiuretic hormone secretion (SIADH). However, it has not yet been approved by the National Medical Products Administration (NMPA) of China for the treatment of PKD. Due to the potential occurrence of frequent and severe adverse reactions, such as liver injury, dehydration, and gout, it is clinically used mostly as preparatory treatment before renal transplantation in patients with end-stage kidney disease. At present, there is an urgent clinical need for novel therapeutic drugs for PKD.

### SUMMARY

Objectives: In view of the problem in the prior art that there is a lack of effective clinical treatment for polycystic kidney disease (PKD), the present disclosure provides, for the first time, novel medical use of a PPARα/δ dual agonist in the treatment of PKD.

Another objective of the present disclosure is to provide a pharmaceutical composition for preventing or treating polycystic kidney disease (PKD).

Technical solution: To achieve the above objectives, the present disclosure provides use of a PPARα/δ dual agonist or a pharmaceutically acceptable salt thereof in the preparation of a medicament for preventing or treating polycystic kidney disease (PKD).

Specifically, the PKD includes autosomal dominant (adult) polycystic kidney disease (ADPKD) and autosomal recessive (infantile) polycystic kidney disease (ARPKD).

Specifically, the PPARα/δ dual agonist includes any one of the compounds protected in the following literature references or patents: *(1)* European Journal of Pharmacology 882 (2020) 173300; (2) Bioorganic Chemistry 99(2020)103803; (3) European Journal of Medicinal Chemistry 218 (2021) 113388; (4) Bioorganic & Medicinal Chemistry 24 (2016) 5455-5461; (5) Bioorganic & Medicinal Chemistry Letters 16 (2006) 554-558; (6) Chemistry & Biology December 1997, 4:909-918, ACS Med. Chem. Lett. 2019, 10, 1068-1073; (7) PCT patent: PCTCN2022072221); (8) PCT patent: PCTCN2022141359; and (9) PCT patent: PCTCN2024072800.

Specifically, the PPARα/δ dual agonist is selected from any one of the following compounds:

| Compound No. | Compound structure |
|---|---|
| 1 | 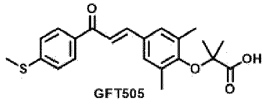 |
| 2 | 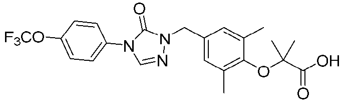 |
| 3 | 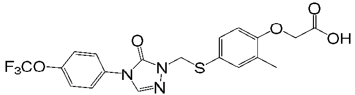 |
| 4 | 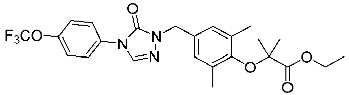 |
| 5 | 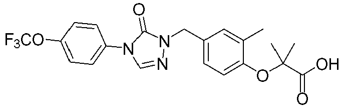 |
| 6 | 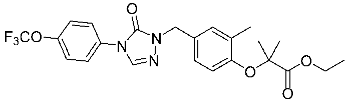 |
| 7 | 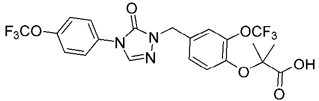 |
| 8 | 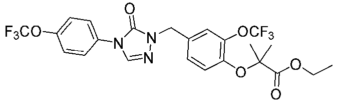 |
| 9 | 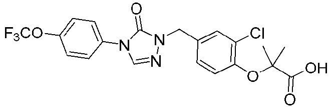 |
| 10 | 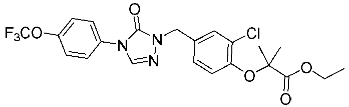 |
| 11 | 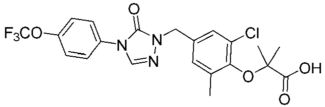 |
| 12 | 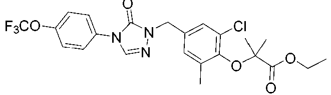 |
| 13 | 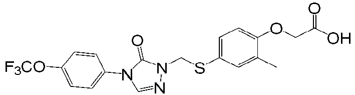 |
| 14 | 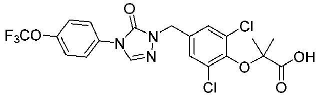 |
| 15 | 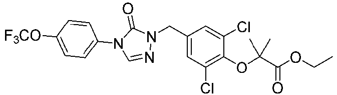 |
| 16 | 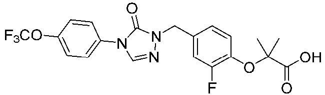 |
| 17 | 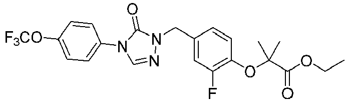 |
| 18 | 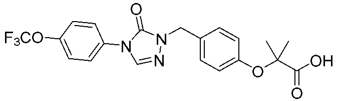 |
| 19 | 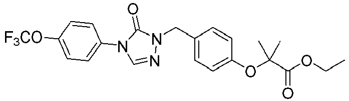 |
| 20 | 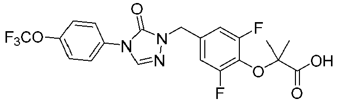 |
| 21 | 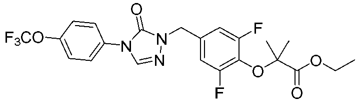 |
| 22 | 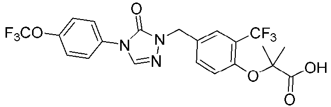 |
| 23 | 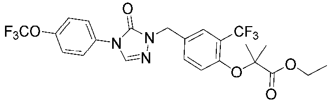 |
| 24 | 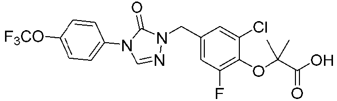 |
| 25 | 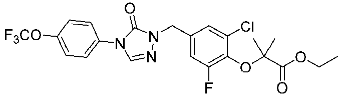 |
| 26 | 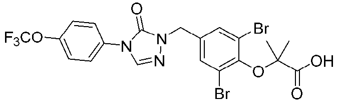 |
| 27 | 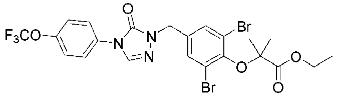 |
| 28 | 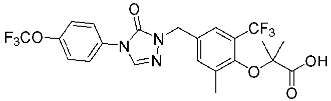 |
| 29 | 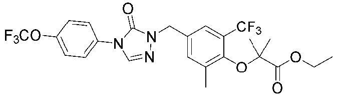 |
| 30 | 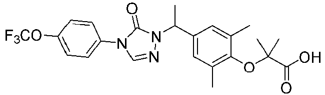 |
| 31 | 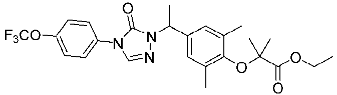 |
| 32 | 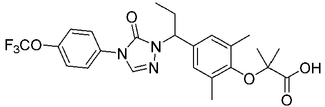 |
| 33 | 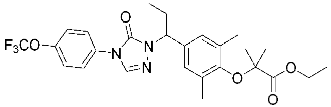 |
| 34 | 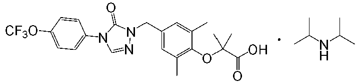 |
| 45 | 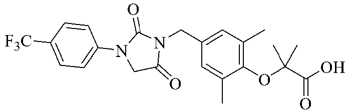 |
| 46 | 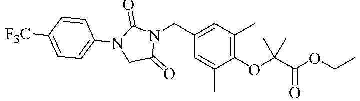 |
| 47 | 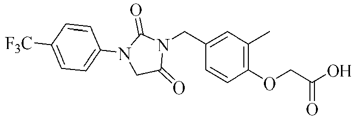 |
| 48 | 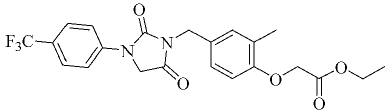 |
| 48 | 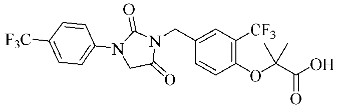 |
| 49 | 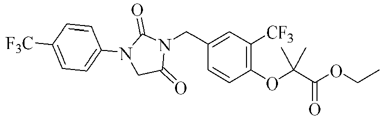 |
| 50 | 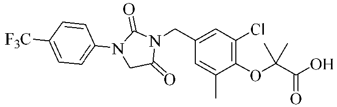 |
| 51 | 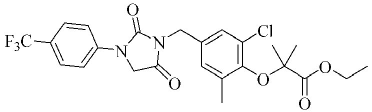 |
| 52 | 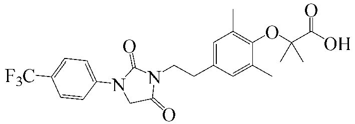 |
| 53 | 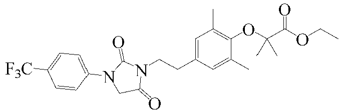 |
| 54 | 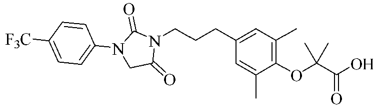 |
| 55 | 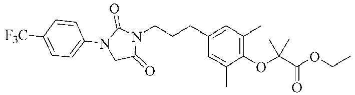 |

Preferably, the PPARα/δ dual agonist includes any one of 2-(2,6-dimethyl-4-((5-oxo-4-(4-(trifluoromethoxy)phenyl)-4,5-dihydro-1H-1,2,4-triazol-1-yl)met hyl)phenoxy)-2-methylpropanoic acid, 2-(4-((2,5-dioxo-3-(4-(trifluoromethyl)phenyl)imidazolidin-1-yl)methyl)-2,6-dimethylphenoxy)-2-methylpropanoic acid, and 2-[2,6-dimethyl-4-[(1E)-3-[4-(methylthio)phenyl]-3-oxo-1-propen-1-yl]phenoxy]-2-methylpropa noic acid.

Specifically, the PPARα/δ dual agonist may be used in the form of a pharmaceutically acceptable salt, and the salt includes an inorganic salt (metal salt and non-metal salt), an organic salt (acidic or basic organic substance), and a complex salt (compounds containing both acidic groups and basic groups).

Specifically, the PPARα/δ dual agonist includes dosage forms acceptable for medical, pharmaceutical, health product, and food use, as well as compound dosage forms containing the compound, including injectable dosage forms, oral dosage forms, and topical dosage forms.

Further, the injectable dosage forms include injection solutions, lyophilized powders for injection, powders for injection, injectable microspheres, liposomal injections, solutions, emulsions, suspensions, injectable nano-formulations, and compatible infusion vehicles therefor; the oral dosage forms include immediate-release tablets, sustained-release or controlled-release tablets, immediate-release capsules, sustained-release or controlled-release capsules, granules, powders, bulk powders, micropellets, microspheres, effervescent preparations, pastes, soft capsules, immediate-release preparations, mixtures, oral solution dosage forms, and oral nano-formulations; and the topical dosage forms include topical sprays, oral sprays, inhalants, tinctures, effervescent formulations, suppositories, patches, ointments, gels, plasters, films, pastes, drops, liniments, and topical nano-formulations.

The pharmaceutical composition of the present disclosure for preventing or treating polycystic kidney disease (PKD) includes, as an active ingredient, a PPARα/δ dual agonist or a pharmaceutically acceptable salt thereof, and a pharmaceutically acceptable excipient and a carrier composition.

Specifically, the polycystic kidney disease includes autosomal dominant polycystic kidney disease (ADPKD) and autosomal recessive polycystic kidney disease (ARPKD).

Specifically, the dosage form of the composition containing the compound together with the excipient and carrier is a capsule, a powder, a tablet, a granule, a pill, an injection, a syrup, an oral solution, an inhalant, an ointment, a suppository, or a patch.

Based on the different gene regulatory effects and tissue distribution of the three PPAR subtypes, the present disclosure, for the first time, uses compounds that simultaneously and selectively activate both PPAR-α and δ(β) receptors, 2-(2,6-dimethyl-4-((5-oxo-4-(4-(trifluoromethoxy)phenyl)-4,5-dihydro-1H-1,2,4-triazol-1-yl)met hyl)phenoxy)-2-methylpropanoic acid, 2-(4-((2,5-dioxo-3-(4-(trifluoromethyl)phenyl)imidazolidin-1-yl)methyl)-2,6-dimethylphenoxy)-2-methylpropanoic acid, and 2-[2,6-dimethyl-4-[(1E)-3-[4-(methylthio)phenyl]-3-oxo-1-propen-1-yl]phenoxy]-2-methylpropa noic acid, to evaluate therapeutic efficacy in PKD animal models. These compounds exhibit very good efficacy against PKD, strongly suggesting that such compounds have broad prospects for the treatment of PKD.

Specifically, the compound includes 2-(2,6-dimethyl-4-((5-oxo-4-(4-(trifluoromethoxy)phenyl)-4,5-dihydro-1H-1,2,4-triazol-1-yl)met hyl)phenoxy)-2-methylpropanoic acid (DA002S), and a chemical structural formula thereof is shown as follows:

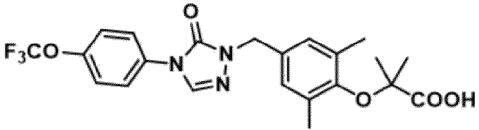

Specifically, the compound includes 2-(4-((2,5-dioxo-3-(4-(trifluoromethyl)phenyl)imidazolidin-1-yl)methyl)-2,6-dimethylphenoxy)-2-methylpropanoic acid (DA002S/V1), and a chemical structural formula thereof is shown as follows:

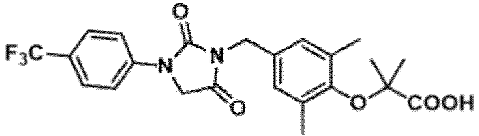

Specifically, the compound includes 2-[2,6-dimethyl-4-[(1E)-3-[4-(methylthio)phenyl]-3-oxo-1-propen-1-yl]phenoxy]-2-methylpropa noic acid (GFT505), and a chemical structural formula thereof is shown as follows:

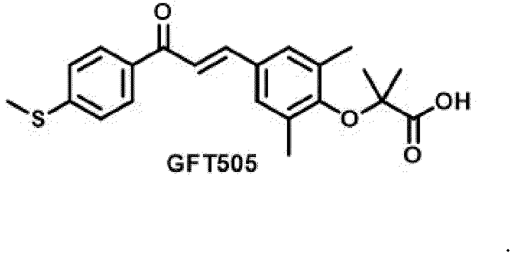

In the present disclosure, homozygous kidney-specific Pkd1 gene knockout mice, , generated by crossing Pkd1^{flox/flox} mice with Ksp-Cre mice followed by intercrossing of the offspring, are one of the internationally recognized classical animal models for PKD, enabling the rapid assessment of drug efficacy and toxicity regarding PKD. Tolvaptan (TVP) was used as a positive drug control group. The results demonstrated that DA002S and DA002S/V1 exhibited stronger activity.

Beneficial effects: compared with the prior art, the present disclosure has the following advantages:

The present disclosure, for the first time, provides use of a PPARα/δ dual agonist in the treatment of PKD, offering a completely new option for the treatment of PKD, and has broad therapeutic prospects. It is demonstrated by *in vivo* experiments in the present disclosure that, when compared with the model vehicle control group, the mice treated with TVP, DA002S, DA002S/V1, and GFT505 had smaller kidney volumes, indicating better therapeutic effects. In addition, the compounds DA002S, DA002S/V1, and GFT505 all exhibited therapeutic effects comparable to those of the positive drug TVP. H&E staining of kidney tissue sections revealed that, compared with the control group, mice treated with TVP, DA002S, DA002S/V1, and GFT505 exhibited a significant reduction in both the number and area of cysts. The above results indicate that DA002S, DA002S/V1, and GFT505 have good therapeutic effects.

In the acute ADPKD mouse administration model, compared with the model vehicle control group, after treatment with TVP (1 mg/kg), DA002S (1 mg/kg), DA002S/V1 (0.1 mg/kg), and GFT505 (10 mg/kg), the mouse kidney volumes were reduced, and the kidney weight indexes were significantly decreased. In addition, the renal cystic indexes (cyst area) of mice after administration were significantly reduced, indicating that cyst development was inhibited. Specifically, the *in-vivo* efficacy of DA002S was superior to that of tolvaptan at the same dose, and the efficacy of DA002S/V1 at a lower dose was significantly superior to that of tolvaptan at a higher dose. In conclusion, DA002S, DA002S/V1, and GFT505 demonstrated the ability to inhibit the progress of ADPKD and improve renal function, and exhibit good *in-vivo* efficacy against ADPKD. In addition, the compounds of the present disclosure also exhibit good therapeutic effects against ARPKD.

Therefore, the present disclosure, for the first time, discovers that a PPARα/δ dual agonist or a pharmaceutically acceptable salt thereof can significantly improve the polycystic kidney phenotype in acute ADPKD/ARPKD model mice, and thus can be used for preparing drugs for preventing or treating polycystic kidney disease (PKD).

### BRIEF DESCRIPTION OF THE DRAWINGS

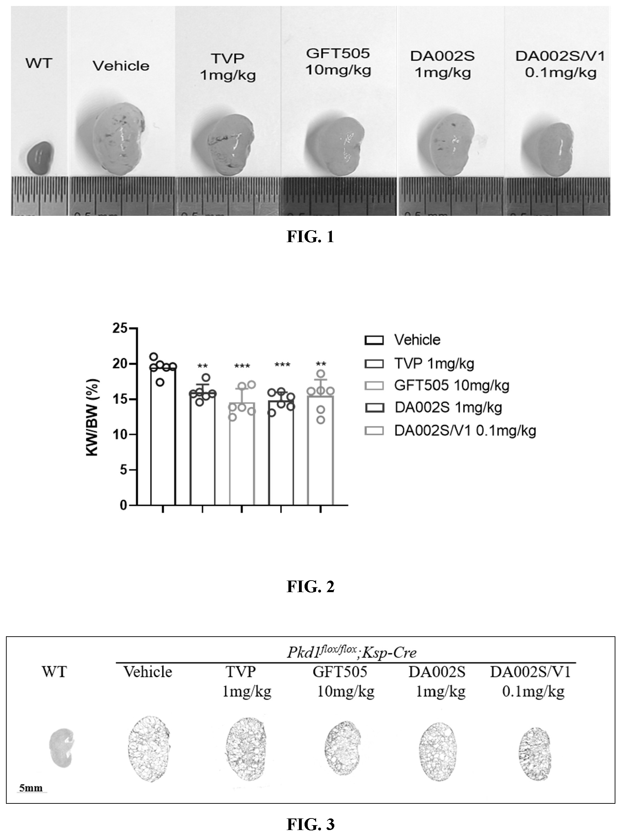

FIG. 1 is a photograph of intact kidneys collected at the time of dissection at the end of dosing from representative animals in a wild-type (WT) mouse group, an ADPKD model vehicle control group, a TVP (1 mg/kg) treatment group, a DA002S (1 mg/kg) treatment group, a DA002S/V1 (0.1 mg/kg) treatment group, and a GFT505 (10 mg/kg) treatment group.
FIG. 2 is a statistical bar chart of kidney indices (combined weight of both kidneys divided by body weight) of mice in an ADPKD model vehicle control group, a TVP (1 mg/kg) treatment group, a DA002S (1 mg/kg) treatment group, a DA002S/V1 (0.1 mg/kg) treatment group, and a GFT505 (10 mg/kg) treatment group. Mean ± SD, n = 6. Compared with the vehicle group, ****P <0.001, **P <0.01.*
FIG. 3 shows H&E staining of kidney sections from mice in an ADPKD model vehicle control group, a TVP (1 mg/kg) treatment group, a DA002S (1 mg/kg) treatment group, a DA002S/V1 (0.1 mg/kg) treatment group, and a GFT505 (10 mg/kg) treatment group.

### DETAILED DESCRIPTIONS OF THE EMBODIMENTS

The present disclosure is further described in detail through the following examples. In the present disclosure, the following examples are provided for better illustrating the present disclosure and are not intended to limit the scope of the present disclosure.

In the examples, 2-(2,6-dimethyl-4-((5-oxo-4-(4-(trifluoromethoxy)phenyl)-4,5-dihydro-1H-1,2,4-triazol-1-yl)met hyl)phenoxy)-2-methylpropanoic acid, 2-(4-((2,5-dioxo-3-(4-(trifluoromethyl)phenyl)imidazolidin-1-yl)methyl)-2,6-dimethylphenoxy)-2-methylpropanoic acid, and 2-[2,6-dimethyl-4-[(1E)-3-[4-(methylthio)phenyl]-3-oxo-1-propen-1-yl]phenoxy]-2-methylpropa noic acid were all synthesized according to methods in the prior art.

Homozygous kidney-specific *Pkd1* gene knockout mice were obtained by crossing Pkd1^{flox/flox} mice with *Ksp-Cre* mice, followed by intercrossing of the offspring. The model mice developed renal cysts in an embryonic stage and rapidly progressed to renal failure, and were used as an acute ADPKD mouse model. The construction method thereof was performed with reference to the method described in Hum Mol Genet.2008 Jun 1;17(11):1505-16.

### Example 1

Homozygous kidney-specific *Pkd1* gene knockout mice were obtained by crossing Pkd1^{flox/flox} mice with *Ksp-Cre* mice, followed by intercrossing of the offspring. The model mice developed renal cysts in an embryonic stage and rapidly progressed to renal failure, and were used as an acute ADPKD mouse model.

The drug administration experiment using the acute ADPKD mouse model was divided into five groups, that is, a model vehicle control group, a group treated with 2-(2,6-dimethyl-4-((5-oxo-4-(4-(trifluoromethoxy)phenyl)-4,5-dihydro-1H-1,2,4-triazol-1-yl)met hyl)phenoxy)-2-methylpropanoic acid (DA002S, 1 mg/kg), 2-(4-((2,5-dioxo-3-(4-(trifluoromethyl)phenyl)imidazolidin-1-yl)methyl)-2,6-dimethylphenoxy)-2-methylpropanoic acid (DA002S/V1, 0.1 mg/kg), and 2-[2,6-dimethyl-4-[(1E)-3-[4-(methylthio)phenyl]-3-oxo-1-propen-1-yl]phenoxy]-2-methylpropa noic acid (GFT505, 10 mg/kg), and a positive drug control group treated with tolvaptan (TVP, 1 mg/kg), with 6 mice in each group. Subcutaneous administration was initiated on postnatal day 6, once daily for 7 consecutive days. After the experiment was completed on day 12, the kidney weight index (combined weight of both kidneys/body weight) and kidney tissue morphology (H&E staining of tissue sections) of the mice were analyzed and compared, thereby evaluating the effects of DA002S, DA002S/V1, and GFT505 on improving the polycystic kidney phenotype of mice in the acute ADPKD model. The results are shown in FIGS. 1-3.

The results showed that, compared with the model vehicle control group, mice treated with TVP, DA002S, DA002S/V1, and GFT505 had smaller kidney volumes, indicating better therapeutic effects (FIG. 1). Especially, mice treated with the low dose of DA002S/V1 had the smallest kidney volume, proving a more significant effect, and the effect was significantly superior to that of the high-dose positive control drug TVP. Analysis of the kidney weight index of mice revealed that, compared with the control group, the kidney weight indexes of mice treated with TVP, DA002S, DA002S/V1, and GFT505 were all reduced (**P < 0.01, *** P < 0.001 VS. Vehicle group) (FIG. 2). In addition, the compounds DA002S, DA002S/V1, and GFT505 all exhibited therapeutic effects comparable to those of the positive drug TVP. H&E staining of kidney tissue sections revealed that, compared with the control group, mice treated with TVP, DA002S, DA002S/V1, and GFT505 exhibited a significant reduction in both the number and area of cysts (FIG. 3). Especially, mice treated with the low dose of DA002S/V1 had the smallest number and area of cysts, proving a more significant effect, and the effect was significantly superior to that of the high-dose positive control drug TVP. The above results indicate that DA002S, DA002S/V1, and GFT505 have good therapeutic effects against ADPKD.

In summary, in the acute ADPKD mouse administration model, compared with the model vehicle control group, after treatment with TVP (1 mg/kg), DA002S (1 mg/kg), DA002S/V1 (0.1 mg/kg), and GFT505 (10 mg/kg), the mouse kidney volumes were reduced, and the kidney weight indexes were significantly decreased. In addition, the renal cystic indexes (cyst area) of mice after administration were significantly reduced, indicating that cyst development was inhibited. Specifically, the *in-vivo* efficacy of DA002S was superior to that of tolvaptan at the same dose, and the efficacy of DA002S/V1 at a lower dose was significantly superior to that of tolvaptan at a higher dose. In conclusion, DA002S, DA002S/V1, and GFT505 demonstrated the ability to inhibit the progress of ADPKD polycystic kidney disease and to improve renal function, and to exhibit good *in-vivo* efficacy against ADPKD. Other compounds of the present disclosure also exhibited similar effects. In addition, the compounds also exhibited good therapeutic effects in an ARPKD model, and can be used in the preparation of drugs for preventing or treating polycystic kidney disease (PKD).

## Claims

1. Use of a PPARα/δ dual agonist or a pharmaceutically acceptable salt thereof in the preparation of a medicament for preventing or treating polycystic kidney disease (PKD).

2. The use according to claim 1, wherein the PKD comprises autosomal dominant (adult) polycystic kidney disease (ADPKD) and autosomal recessive (infantile) polycystic kidney disease (ARPKD).

3. The use according to claim 1, wherein the PPARα/δ dual agonist comprises any one of the compounds protected in the following literature references or patents: *(1) European Journal of Pharmacology* 882 (2020) 173300; (2) *Bioorganic Chemistry* 99(2020)103803; (3) *European Journal of Medicinal Chemistry* 218 (2021) 113388; (4) *Bioorganic & Medicinal Chemistry 24* (2016) 5455-5461; *(5) Bioorganic & Medicinal Chemistry Letters* 16 (2006) 554-558; (6) *Chemistry & Biology* December 1997, 4:909-918, *ACS Med. Chem. Lett.* 2019, 10, 1068-1073; (7) PCT patent: PCTCN2022072221); (8) PCT patent: PCTCN2022141359; and (9) PCT patent: PCTCN2024072800.

4. The use according to claim 1, wherein the PPARα/δ dual agonist is selected from any one of the following compounds:
| Compound No. | Compound structure |
|---|---|
| 1 | 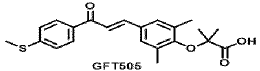 |
| 2 | 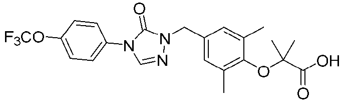 |
| 3 | 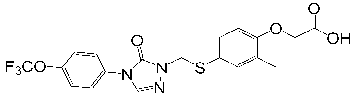 |
| 4 | 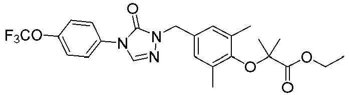 |
| 5 | 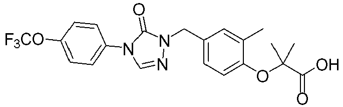 |
| 6 | 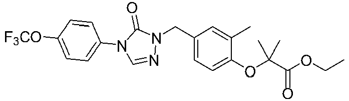 |
| 7 | 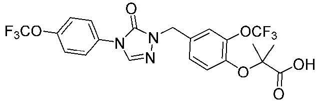 |
| 8 | 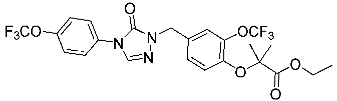 |
| 9 | 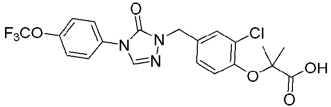 |
| 10 | 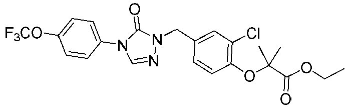 |
| 11 | 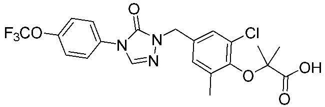 |
| 12 | 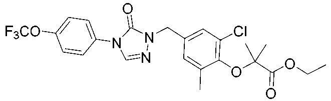 |
| 13 | 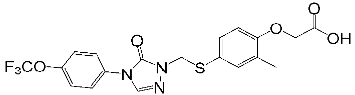 |
| 14 | 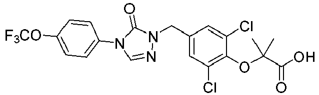 |
| 15 | 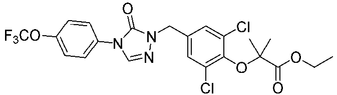 |
| 16 | 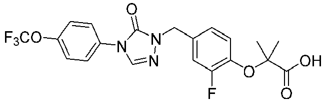 |
| 17 | 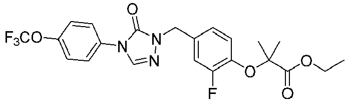 |
| 18 | 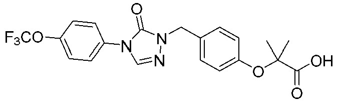 |
| 19 | 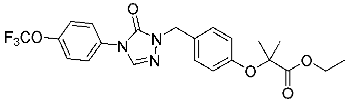 |
| 20 | 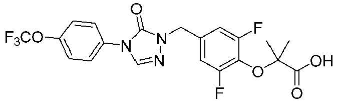 |
| 21 | 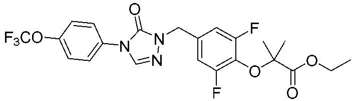 |
| 22 | 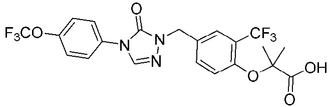 |
| 23 | 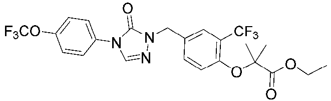 |
| 24 | 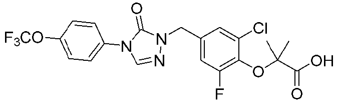 |
| 25 | 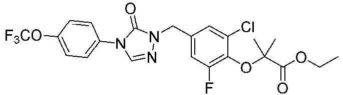 |
| 26 | 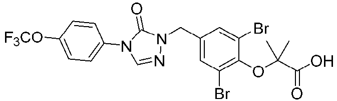 |
| 27 | 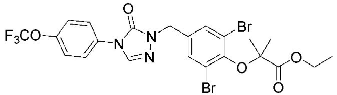 |
| 28 | 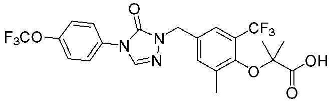 |
| 29 | 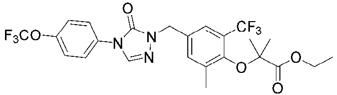 |
| 30 | 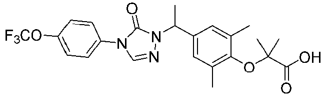 |
| 31 | 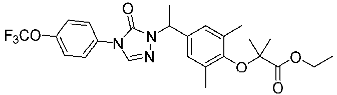 |
| 32 | 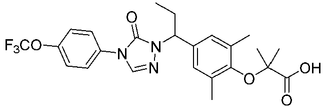 |
| 33 | 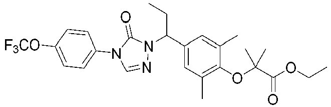 |
| 34 | 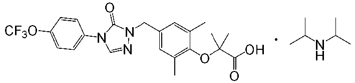 |
| 45 | 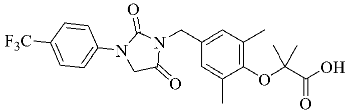 |
| 46 | 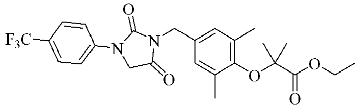 |
| 47 | 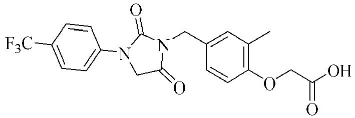 |
| 48 | 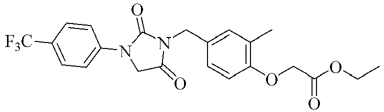 |
| 48 | 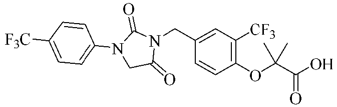 |
| 49 | 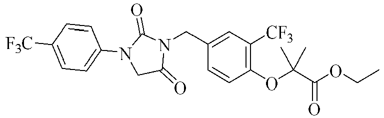 |
| 50 | 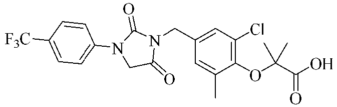 |
| 51 | 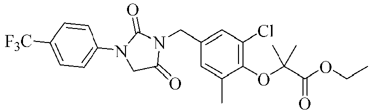 |
| 52 | 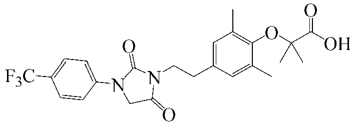 |
| 53 | 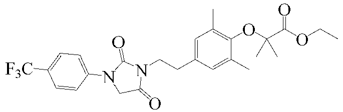 |
| 54 | 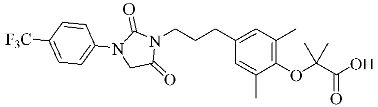 |
| 55 | 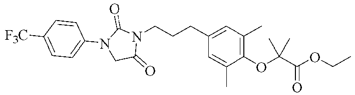 |

5. The use according to claim 1, wherein the PPARα/δ dual agonist comprises any one of 2-(2,6-dimethyl-4-((5-oxo-4-(4-(trifluoromethoxy)phenyl)-4,5-dihydro-1H-1,2,4-triazol-1-yl)met hyl)phenoxy)-2-methylpropanoic acid, 2-(4-((2,5-dioxo-3-(4-(trifluoromethyl)phenyl)imidazolidin-1-yl)methyl)-2,6-dimethylphenoxy)-2-methylpropanoic acid, and 2-[2,6-dimethyl-4-[(1E)-3-[4-(methylthio)phenyl]-3-oxo-1-propen-1-yl]phenoxy]-2-methylpropa noic acid.

6. The use according to claim 1, wherein the PPARα/δ dual agonist is used in the form of a pharmaceutically acceptable salt, and the salt comprises an inorganic salt, an organic salt, and a complex salt.

7. The use according to claim 1, wherein the PPARα/δ dual agonist comprises dosage forms acceptable for medical, pharmaceutical, health product, and food use, as well as compound dosage forms containing the compound, comprising injectable dosage forms, oral dosage forms, and topical dosage forms.

8. The use according to claim 6, wherein the injectable dosage forms comprise injection solutions, lyophilized powders for injection, powders for injection, injectable microspheres, liposomal injections, solutions, emulsions, suspensions, injectable nano-formulations, and compatible infusion vehicles therefor; the oral dosage forms comprise immediate-release tablets, sustained-release or controlled-release tablets, immediate-release capsules, sustained-release or controlled-release capsules, granules, powders, bulk powders, micropellets, microspheres, effervescent preparations, pastes, soft capsules, immediate-release preparations, mixtures, oral solution dosage forms, and oral nano-formulations; and the topical dosage forms comprise topical sprays, oral sprays, inhalants, tinctures, effervescent formulations, suppositories, patches, ointments, gels, plasters, films, pastes, drops, liniments, and topical nano-formulations.

9. A pharmaceutical composition for preventing or treating polycystic kidney disease (PKD), comprising, as an active ingredient, a PPARα/δ dual agonist or a pharmaceutically acceptable salt thereof, and a pharmaceutically acceptable excipient and a drug delivery system.

10. The pharmaceutical composition according to claim 9, wherein the PKD comprises autosomal dominant polycystic kidney disease (ADPKD) and autosomal recessive polycystic kidney disease (ARPKD).
